# FASCICULE DE BREVET EUROPEEN

(11) **EP 1 303 246 B1**
(45) Date de publication et mention de la délivrance du brevet: **27.02.2008**
(21) Numéro de dépôt: 01953198.7
(22) Date de dépôt: 09.07.2001
(51) Int. Cl.: A61K 8/31, A61K 8/81, A61K 8/90, A61Q 1/02, A61Q 1/12, A61Q 3/02

(54) **COMPOSITION COSMETIQUE LONGUE TENUE COMPRENANT UN MATERIAU PRO-ADHESIF PARTICULIER**
HALTBARE KOSMETISCHE ZUSAMMENSETZUNG, DIE EIN BESTIMMTES HAFTUNGSFÖRDERNDES MATERIAL ENTHÄLT
LONG-LASTING COSMETIC COMPOSITION COMPRISING A SPECIFIC PRO-ADHESIVE MATERIAL

(30) Priorité: 13.07.2000 FR 0009217
(43) Date de publication de la demande: 23.04.2003
(73) Titulaire: L'ORÉAL, 75008 Paris (FR)
(72) Inventeur: BERNARD, Pascale, F-94370 Sucy-en-Brie (FR); HADASCH, Anke, F-75005 Paris (FR); TOUMI, Béatrice, F-91370 Verrières le Buisson (FR)
(74) Mandataire: Kromer, Christophe
(86) Numéro de dépôt international: PCT/FR2001/002206
(87) Numéro de publication internationale: WO 2002/005760

(56) Documents cités:
- EP-A- 0 600 445
- EP-A- 0 615 744
- WO-A-96/36308
- WO-A-98/42298
- FR-A- 2 795 079
- US-A- 5 385 729
- US-A- 5 756 437
- US-A- 5 876 744

## Description

La présente invention se rapporte à un kit de maquillage comprenant une composition comprenant un matériau pro-adhésif. Plus spécialement, l'invention se rapporte à un kit de longue tenue et sans transfert pour le maquillage de la peau aussi bien du visage que du corps humain et/ou des phanères.

Les compositions cosmétiques, notamment de maquillage telles que les rouges à lèvres, les fonds de teint, les produits de maquillage du corps, les anticernes, les fards à paupières ou les poudres, comprennent généralement des corps gras tels que des huiles et/ou des cires, et une phase particulaire généralement composée de charges et de pigments. Elles peuvent ainsi se présenter sous la forme d'un gel anhydre, sous forme de stick ou bâton ou sous forme de pâte souple, comme par exemple certains fonds de teint, fards à paupières ou rouges à lèvres. Elles peuvent encore se présenter sous la forme d'une poudre, qui peut être par exemple libre, compactée ou pressée. Les compositions de maquillage peuvent également comprendre de l'eau ou une phase hydrophile, et se présenter alors notamment sous forme d'émulsion huile-dans-eau, eau-dans-huile, émulsion multiple, notamment lorsqu'il s'agit d'un fond de teint, de crème teintée, de crème de soin ou d'un produit solaire. Les vernis à ongles se présentent généralement sous la forme d'une solution d'un solvant organique.

Il est connu du document EP-A-600445) des compositions cosmétiques de maquillage présentant une bonne résistance à l'eau, à l'huile et au sébum, se présentant sous forme d'émulsions eau-dans-huile comprenant de 1 à 54 % en poids d'une résine soluble dans la phase externe et un agent de coloration dans la phase interne:

Ces compositions, lorsqu'elles sont appliquées sur la peau, les muqueuses ou les semi-muqueuses, ne présentent pas toujours une bonne tenue. On a en effet constaté que certaines compositions avaient tendance à se propager à l'intérieur des ridules et/ou des rides de la peau, dans le cas des fonds de teint; dans les ridules qui entourent les lèvres, dans le cas des rouges à lèvres; dans les plis de la paupière, dans le cas des fards à paupières. On a également constaté, dans le cas notamment des fards à paupières, l'apparition de stries dans le maquillage, générées par les mouvements des paupières. De même, au cours du temps, il arrive que la couleur d'origine vire. Dans le cas des vernis à ongles, il arrive que le produit se craquèle, s'écaille ou ne résiste pas aux frottements.

Par ailleurs, certains produits de maquillage peuvent présenter l'inconvénient de transférer. On entend par là que la composition est susceptible de se déposer, au moins en partie, sur certains supports avec lesquels elle est mise en contact, tels que, par exemple, un verre, un vêtement ou la peau.

En se déposant, ladite composition laisse une trace sur ledit support. Il s'en suit donc une persistance médiocre de la composition sur la peau ou les muqueuses, d'où la nécessité de renouveler régulièrement son application.

Par ailleurs, l'apparition de traces inacceptables sur certains vêtements et notamment sur les cols de chemisier peut écarter certaines femmes de l'utilisation de ce type de maquillage.

Tous ces phénomènes engendrent un effet inesthétique que l'on souhaite bien évidemment éviter.

Il subsiste donc le besoin de produits, notamment de maquillage, qui présentent une bonne tenue tout en possédant de bonnes propriétés cosmétiques, notamment des propriétés de glissant à l'application, ainsi que des qualités de douceur, d'hydratation et de confort après maquillage.

La demanderesse a constaté, de façon tout à fait surprenante, que l'utilisation d'un matériau pro-adhésif particulier dans une composition cosmétique ou physiologique permettait d'obtenir un maquillage de très bonne tenue, ne transférant pas du tout, résistant à l'eau, la composition étant par ailleurs aisément délitable.

Un premier objet de la présente invention est un kit de maquillage comprenant :
- i°) une composition dite associable comprenant, dans un support cosmétiquement acceptable, au moins un matériau pro-adhésif répondant à la condition suivante :
   - 10⁸ Pa ≥ G'(35°C) ≥ 2 10⁴ Pa pour toute fréquence allant de 2 10⁻² à 2 Hz, de préférence :
      - 10⁷ Pa ≥ G'(35°C) ≥ 2 10⁴ Pa pour toute fréquence allant de 2 10⁻² à 2 Hz,
   dans laquelle :
   - G'(35°C) est le module élastique de cisaillement dudit matériau pro-adhésif, mesuré à la température de 35°C,
   ledit matériau pro-adhésif étant choisi parmi les polyisobutylènes,
   et,
- ii°) une « composition associée » comprenant, dans un support cosmétiquement acceptable, au moins un composé diffusant choisi parmi les huiles de masse moléculaire inférieure ou égale à 20000 tel que le système adhésif « matériau pro-adhésif + composé diffusant » répond aux conditions suivantes :
   - G'_{SA}(2 Hz, 35°C) ≥ 10³ Pa,
   - 10⁸ Pa ≥ G'_{SA}(35°C), de préférence 10⁷ Pa ≥ G'_{SA}(35°C), et
   - G'_{SA}(2 10⁻² Hz, 35°C) ≤ 3 10⁵ Pa,
   dans lesquelles :
   - G'_{SA}(2 Hz, 35°C) est le module élastique de cisaillement du système adhésif, mesuré à la fréquence de 2 Hz et à la température de 35°C,
   - G'_{SA}(35°C) est le module élastique de cisaillement du système adhésif, à la température de 35°C, pour toute fréquence comprise entre 2 10⁻² et 2 Hz
   - G'_{SA}(210⁻² Hz, 35°C) est le module élastique de cisaillement du système adhésif, mesuré à la fréquence de 2 10⁻² Hz et à la température de 35°C.

La composition associable du kit selon l'invention présente d'excellentes propriétés de tenue sur la peau et de « non transfert ». Elle adhère bien à la peau. Elle est résistante au frottement, à la sueur, au sebum, à l'eau. Elle se délite facilement. Elle permet un dépôt homogène dans le temps sur la peau et un maquillage uniforme. Une fois appliquée sur la peau, elle ne migre pas et présente une très bonne tenue de la couleur dans le temps. La composition s'étale facilement et est très confortable. Elle est agréable à l'application et à porter tout au long de la journée. Elle est non grasse et ne brille pas au cours du temps. Elle présente un toucher onctueux et agréable.

Un autre objet de l'invention est l'utilisation dans ou pour la fabrication d'une composition cosmétique ou physiologique pulvérulente, d'au moins un matériau pro-adhésif tel que défini ci-dessus pour diminuer, voire supprimer, le transfert et/ou la migration et/ou augmenter la tenue de la composition déposée sur les lèvres et/ou la peau d'être humain vers un support mis en contact avec le dépôt.

Un autre objet de l'invention est l'utilisation dans ou pour la fabrication d'une composition cosmétique ou physiologique pulvérulente, d'au moins un matériau pro-adhésif tel que défini ci-dessus pour augmenter la tenue de la couleur de la composition dans le temps.

L'invention a encore pour objet un procédé non thérapeutique de maquillage de la peau, des muqueuses, des semi-muqueuses et/ou des phanères d'humain, consistant à appliquer sur lesdites peau, muqueuses, semi-muqueuses et/ou phanères d'humain la composition associée avant ou après la composition dite associable du kit selon l'une des revendications précédentes.

Par composition pulvérulente ou sous forme pulvérulente, on entend selon l'invention une composition qui comprend au moins un composé pulvérulent. Les composés pulvérulents peuvent être choisis parmi les pigments et/ou les nacres et/ou les charges et/ou leurs mélanges habituellement utilisés dans les compositions cosmétiques. Avantageusement, les composés pulvérulents sont présents à une teneur allant de 60 à 99,9%, de préférence encore de 80 à 99,9% en poids, par rapport au poids total de la composition.

Par composition physiologique, on entend selon l'invention une composition physiologiquement acceptable non toxique et susceptible d'être appliquée sur la peau, les phanères ou les lèvres d'êtres humains. Par composition cosmétique, on entend au sens de l'invention une composition d'aspect, d'odeur et de toucher agréables.

Dans un mode particulier de réalisation de l'invention, la composition associable comprend au moins 80% de composés pulvérulents. On obtient alors un produit présentant une texture particulière, sous forme de gomme c'est-à-dire d'un produit solide, qui ne se déforme pas sous son propre poids.

La composition associable du kit selon l'invention comprend en outre au moins un matériau pro-adhésif. Par matériau, on entend au sens de la présente invention un polymère ou un système polymérique, de préférence au moins en partie non réticulé, de façon encore plus préférée pratiquement non réticulé, pouvant comprendre un ou plusieurs polymères de natures différentes. Par pro-adhésif, on entend au sens de la présente invention susceptible de devenir adhésif généralement en interaction avec un composé diffusant associé ou en interaction avec les exsudats biologiques. Par adhésif, on entend au sens de la présente invention qui adhère à une surface, c'est-à-dire que, après contact entre l'index et la surface de la composition dans laquelle est compris le matériau, on ressent une impression de collant de la surface lorsqu'on rompt le contact avec celle-ci en soulevant l'index, de la même manière que ce que l'on peut ressentir après contact avec une face adhésive par exemple d'un ruban adhésif. Après contact sur la peau, la composition associable comprenant le matériau pro-adhésif va généralement coller à la surface de la peau, et donc être de bonne tenue, ou va adhérer à une composition dite associée comprenant au moins un composé diffusant, déposée avant et/ou après la composition associable du kit selon l'invention ; ainsi le produit cosmétique résultant sur la peau ne va généralement pas présenter de collant superficiel, c'est-à-dire que, après contact entre l'index et la surface du produit, on ne ressent pas une impression de collant de la surface lorsqu'on rompt le contact avec celle-ci en soulevant l'index.

Le matériau pro-adhésif selon l'invention peut éventuellement présenter un certain pouvoir collant utilisé seul. Après application de la composition associable du kit selon l'invention sur la peau, en particulier sur le visage, on obtient une composition qui présente une très bonne tenue, grâce à des interactions avec les exsudats biologiques, qui peuvent évoluer au cours du temps. Les exsudats biologiques sont généralement les liquides physiologiques et les excrétions cutanées, notamment le sébum, la sueur, les larmes et la salive. Il est connu de l'homme de l'art que suivant la localisation anatomique, les taux et la nature de ces excrétions varient au cours de la journée.

Le matériau pro-adhésif est utilisé en association avec un composé diffusant qui, après diffusion au sein du réseau polymérique du matériau pro-adhésif, développe un pouvoir collant supérieur à celui du matériau initial : on obtient ainsi un système « matériau pro-adhésif + composé diffusant » qui présente des propriétés adhésives. Le composé diffusant peut par exemple être le sébum ou la sueur sécrétés par la peau au cours du temps. Une composition comprenant le matériau pro-adhésif selon l'invention voit ainsi sa tenue augmenter avec le temps et la libération de sébum ou de sueur par la peau.

Le composé diffusant est présent au sein d'une deuxième composition, appelée ci-après et dans toute la présente demande « composition associée », que l'on applique distinctement et en association avec la composition selon l'invention comprenant le matériau pro-adhésif. La composition associée peut indifféremment être appliquée sur la peau et/ou les phanères avant ou après la composition associable du kit selon l'invention.

Les kits selon l'invention trouvent notamment une application particulièrement intéressante dans le domaine du maquillage de la peau, des muqueuses, des semi-muqueuses, et des phanères. On entend notamment par muqueuse, la partie interne de la paupière inférieure; parmi les semi-muqueuses, on entend plus particulièrement les lèvres du visage; par phanères, on entend les cils, sourcils, cheveux et ongles. Ainsi, l'invention trouve une application toute particulière dans le domaine des produits de maquillage des lèvres du visage et de la peau, tels que les fonds de teint, les anticernes, les fards à paupières, les poudres, les produits de maquillage du corps, les rouges à lèvres, et dans le, domaine des produits de maquillage des phanères (ou de leurs substituts comme les faux-ongles ou les faux cils) tels que les mascaras, les vernis à ongles.

Les propriétés viscoélastiques d'un matériau sont classiquement définies par deux valeurs caractéristiques qui sont les suivantes :
- le module élastique de cisaillement qui représente le comportement élastique du matériau pour une fréquence donnée et qui est classiquement noté G',
- le module visqueux de cisaillement qui représente le comportement visqueux du matériau pour une fréquence donnée et qui est classiquement noté G".

Ces grandeurs sont notamment définies dans le « Handbook of Pressure Sensitive Adhesive Technology » 3rd edition, D. Satas, chap.9 p.155 à 157.

Ces propriétés viscoélastiques sont mesurées lors d'essais dynamiques sous sollicitations sinusoïdales de faible amplitude (petites déformations) réalisés à 35°C sur une plage de fréquence allant de 2 10⁻² à 20 Hz sur un Rhéomètre (par exemple de type Haake RS50 ou RS75) sous une sollicitation de torsion/cisaillement, par exemple en géométrie cône-plan (par exemple avec un angle du cône de 1 °).

Lorsque les matériaux sont trop cohésifs pour que l'homme du métier puisse les caractériser sur le rhéomètre de type Haake RS50 ou RS75 ci-dessus (par exemple quand G'_{SA}(2 10⁻² Hz, 35°C) > 10⁸Pa), les propriétés viscoélastiques des matériaux sont mesurées lors d'essais dynamiques sous sollicitations sinusoïdales de faible amplitude (petites déformations) réalisés à 35°C sur une plage de fréquence allant de 2 10⁻² à 20 Hz sur un viscoélasticimètre par exemple de type DMA 2980 de T.A. Instruments en traction sur film. Pour cela, les matériaux sont mis en solution dans un solvant volatil approprié (par exemple à une concentration de 10%) puis coulés sous forme de film dans une matrice téflonnée ; ils sont ensuite mis à sécher à 35°C pour que le solvant volatil s'évapore et que l'on récupère un film formé de ces matériaux. Dans ce cas, on détermine le module élastique en traction E' et le module visqueux en traction E" et on déduit les valeurs G' et G" par les relations G' = E'/3 et G" = E"/3.

Les matériaux pro-adhésifs utilisables selon la présente invention répondent à la condition suivante :
- 10⁸ Pa ≥ G'(35°C) ≥ 2 10⁴, de préférence 10⁷ Pa ≥ G'(35°C) ≥ 2 10⁴, pour toute fréquence allant de 2 10⁻² à 2 Hz,
dans laquelle :
- G'(35°C) est le module élastique de cisaillement dudit matériau pro-adhésif, mesuré à la température de 35°C.

Les matériaux pro-adhésifs selon l'invention peuvent être choisis parmi les adhésifs de type « Pressure Sensitive Adhesives » (adhésifs sensibles à la pression) par exemple, comme ceux cités dans le « Handbook of Pressure Sensitive Adhesive Technology » 3^{rd} édition, D. Satas.

Les matériaux pro-adhésifs selon l'invention sont de préférence des polymères adhésifs choisis parmi les copolymères blocs ou statistiques comprenant au moins un monomère ou une association de monomères dont le polymère résultant a une température de transition vitreuse inférieure à la température ambiante (25°C), ces monomères ou associations de monomères pouvant être choisis parmi le butadiène, l'éthylène, le propylène, l'isoprène, l'isobutylène, une silicone, et leurs mélanges. Des exemples de tels matériaux sont les polymères blocs de type styrène-butadiène-styrène, styrène-(ethylène-butylène)-styrène, styrène-isoprène-styrène comme ceux vendus sous la dénomination commerciale « Kraton » de Shell Chemical Co. ou « Vector » d'Exxon.

Les matériaux pro-adhésifs selon l'invention sont des polymères adhésifs choisis parmi les polyisobutylènes,

Dans une forme particulièrement avantageuse de l'invention, les matériaux pro-adhésifs sont choisis parmi les polyisobutylènes présentant une masse molaire relative Mv supérieure ou égale à 150 000.

Cette masse molaire relative Mv peut être évaluée par sa moyenne viscosimétrique, calculée selon la formule J₀ = 3,06 10⁻² Mv^{0,65}, dans laquelle J₀ est l'indice de Staudinger (en cm³/g). Cet indice est calculé à partir du temps d'écoulement d'une solution de concentration en polyisobutylènes de C = 0,01 g/cm³ dans l'isooctane, à travers un capillaire 1 d'un viscosimètre de type Ubbelhode à 20°C selon la norme ISO 1628.

La masse molaire relative Mv peut également être évaluée par GPC (Gel Permeation Chromatographie) selon le protocole suivant : on injecte 200 µl d'une solution de polymère (matériau adhésif) à 0,5% grâce à une pompe de type « Waters 6000 A », l'éluant étant une solution de 100% THF, débit 1 ml/min, à température ambiante, à travers un jeu de 8 colonnes : µstyragel 500Å + 10⁴Å + 2x10³Å + styragel HR0.5 + 2xHR1 + HR5E (300 x 7,8mm). La détection se fait sur un réfractomètre de type « Waters 410 » et sur un détecteur à UV de type « Waters 490 » à la longueur d'onde 254 nm.

Comme produits commerciaux convenant particulièrement bien à la présente invention, on peut citer les polyisobutylènes de masses molaires relatives Mv supérieure à 150000 de la gamme Oppanol de BASF, notamment les polyisobutylènes de masses molaires relatives Mv respectives 200 000, 400 000 et 1 110 000 vendus sous les dénominations commerciales respectives « Oppanol B 30 SF », « Oppanol B 50 SF » et « Oppanol B 100 » par la société BASF, les polyisobutylènes de masses molaires relative Mv comprises entre 900 000 et 2 200 000 vendus sous la dénomination commerciale « Vistanex MM » par la société Exxon, et leurs mélanges.

Les matériaux pro-adhésifs sont de préférence présents dans la composition associable à une teneur allant de 0,1 à 99%, de préférence de 0,1 à 30%, et de préférence encore de 0,1 à 10% en poids, par rapport au poids total de la composition.

Outre le matériau pro-adhésif défini ci-dessus, la composition associable selon l'invention peut comprendre tout support cosmétiquement acceptable. Par support cosmétiquement acceptable, on entend un milieu compatible avec toutes les matières kératiniques telles que la peau, les ongles, les cheveux, les cils et sourcils, les muqueuses et les semi-muqueuses, et toute autre zone cutanée du corps et du visage, ainsi que tout substitut de ces supports comme les faux-ongles, les faux cils et les prothèses capillaires.

Ce support peut comprendre tout composé hydrosoluble, hydrodispersible, liposoluble, ou lipodispersible cosmétiquement acceptable et classiquement utilisé en cosmétique. Ainsi, le support peut être sous la forme d'une composition pulvérulente, d'une phase grasse anhydre (gel ou solution), sous la forme d'une phase aqueuse (gel ou solution), sous la forme d'une dispersion, d'une solution dans un solvant organique, d'une émulsion H/E ou E/H, ou d'une émulsion multiple, éventuellement stabilisées par un ou plusieurs systèmes organisés.

Par systèmes organisés, on entend au sens de la présente invention des micelles inverses ou des structures « cristal liquide lyotrope » qui sont formées à température ambiante par le mélange de plusieurs tensio-actifs ou le mélange de tensio-actifs et de solvants polaires ou le mélange de plusieurs solvants polaires, les solvants polaires étant par exemple choisis parmi l'eau, le glycérol, le panthénol, le propylène glycol, le butylène glycol et/ou leurs mélanges. L'état cristallin liquide est un état intermédiaire entre l'état solide et l'état liquide. Il est souvent appelé état mésomorphe. Ces systèmes organisés sont thermodynamiquement stables.

Selon sa nature, le matériau pro-adhésif selon l'invention peut se trouver au sein du support sous la forme solubilisée ou sous la forme dispersée, dans une phase aqueuse ou dans une phase anhydre. Le matériau pro-adhésif selon l'invention peut ainsi être sous la forme d'une dispersion aqueuse de particules ou sous la forme d'une dispersion huileuse de particules.

Lorsque la composition associable comprend une phase grasse, celle-ci peut comprendre de préférence au moins une huile cosmétiquement ou physiologiquement acceptable, notamment choisie parmi les huiles d'origine minérale, animale, végétale ou synthétique, carbonées, hydrocarbonées et/ou siliconées, et leurs mélanges, dans la mesure où elles sont compatibles avec l'utilisation envisagée.

On peut également citer les huiles hydrocarbonées telles que l'huile de paraffine ou de vaseline, l'huile de vison, de tortue, de soja, le perhydrosqualène, l'huile d'amande douce, de calophyllum, de palme, de pépins de raisin, de sésame, de maïs, de parléam, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol ; les huiles siliconées telles que les polydiméthylsiloxane (PDMS), éventuellement phénylées telles que les phényltriméthicones, ou éventuellement substitués par des groupements aliphatiques et/ou aromatiques, ou par des groupements fonctionnels tels que des groupements hydroxyle, thiol et/ou amine ; les polysiloxanes modifiés par des acides gras, des alcools gras ou des polyoxyalkylènes, et leurs mélanges.

Avantageusement, on peut utiliser au moins une huile volatile à température ambiante. Par huile volatile, on entend une huile susceptible de s'évaporer de la peau, à température ambiante en moins d'une heure. De préférence, l'huile volatile a une viscosité allant de 0,5 à 25 centistokes à 25°C. Après évaporation de ces huiles, on obtient un dépôt de particules, non collant sur la peau ou les muqueuses. De préférence, on utilise des huiles dont le point éclair est suffisamment élevé pour permettre l'utilisation de ces huiles en formulation. Ces huiles volatiles facilitent, en outre, l'application de la composition sur la peau.

Ces huiles peuvent être des huiles hydrocarbonées, des huiles siliconées comportant éventuellement des groupements alkyle ou alkoxy en bout de chaîne siliconée ou pendante.

Comme huile de silicone volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone. On peut ainsi citer notamment l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane et/ou leurs mélanges.

Comme huile hydrocarbonée volatile, on peut citer les isoparaffines en C₈-C₁₆ telles que l'isododécane, l'isodécane, l'heptane, l'isohexadécane et/ou leurs mélanges.

Ces huiles volatiles peuvent être présentes au sein de la composition associable à une teneur allant de 0,1 à 99% en poids, de préférence de 0,1 à 60%, et encore préférentiellement de 0,1 à 40%, par rapport au poids total de la composition. De préférence, le rapport pondéral d'huile volatile sur le matériau pro-adhésif selon l'invention va de 1 à 20, de préférence encore de 1 à 10, et mieux de 2 à 4.

La phase grasse peut également comprendre au moins une cire, au moins une gomme et/ou au moins un corps gras pâteux, d'origine végétale, animale, minérale ou de synthèse, voire siliconé, et leurs mélanges.

Parmi les cires solides à température ambiante, susceptibles d'être présentes dans la composition selon l'invention, on peut citer les cires hydrocarbonées telles que la cire d'abeilles, la cire de Carnauba, de Candellila, d'Ouricoury, du Japon, les cires de fibres de liège ou de canne à sucre, les cires de paraffine, de lignite, les cires microcristallines, la cire de lanoline, la cire de Montan, les ozokérites, les cires de polyéthylène, les cires obtenues par synthèse de Fischer-Tropsch, les huiles hydrogénées, les esters gras et les glycérides concrets à 25°C. On peut également utiliser des cires de silicone, parmi lesquelles on peut citer les alkyl, alcoxy et/ou esters de polyméthylsiloxane. Les cires peuvent se présenter sous forme de dispersions stables de particules colloïdales de cire telles qu'elles peuvent être préparées selon des méthodes connues, telles que celles de "Micro-emulsions Theory and Practice", L. M. Prince Ed., Academic Press (1977), pages 21-32. Comme cire liquide à température ambiante, on peut citer l'huile de Jojoba.

Les cires peuvent être présentes à raison de 0,1 à 50%, en poids, par rapport au poids total de la composition.

On peut définir les composés gras pâteux à l'aide d'au moins une des propriétés physico-chimiques suivantes :
- une viscosité de 0,1 à 40 Pa.s (1 à 400 poises), de préférence 0,5 à 25 Pa.s, mesurée à 40°C avec un viscosimètre rotatif CONTRAVES TV équipé d'un mobile MS-r3 ou MS-r4 à la fréquence de 240 tours/mn,
- un point de fusion de 25-70°C, de préférence 25-55°C.

Les compositions de l'invention peuvent également comprendre au moins une alkyl, alcoxy ou phényl-diméthicone tels que, par exemple le produit vendu sous la dénomination de "Abil wax 2440" par la Société GOLDSCHMIDT.

Les compositions selon l'invention peuvent également comprendre au moins une résine de silicone comprenant une combinaison des unités R₃SiO_{1/2}, R₂SiO_{2/2}, RSiO_{3/2} et SiO_{4/2}, dans lesquelles R désigne un radical alkyle ayant de 1 à 6 atomes de carbone.

La phase grasse peut en outre contenir au moins un colorant liposoluble. Ce colorant liposoluble est par exemple le rouge Soudan, le DC Red 17, le DC Green 6, le β-carotène, l'huile de soja, le brun Soudan, le DC Yellow, 11, le DC Violet 2, le DC orange 5, le jaune quinoléine, ou leurs mélanges. Ils peuvent représenter de 0,01 à 20 % du poids total de la compositions et mieux de 0,1 à 6 %.

La phase grasse peut représenter de 0,01 à 99,9%, en poids, par rapport au poids total de la composition finale. Lorsque la composition associable est une poudre, par exemple pour le visage, la phase grasse est de préférence présente à une teneur allant de 0,1 à 30% en poids, par rapport au poids total de la composition.

La composition associable selon l'invention peut comprendre au moins une phase de solvant organique, en particulier lorsque la composition est un vernis à ongles.

Comme solvant organique utilisable dans l'invention, on peut citer :
- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les composés cycliques aromatiques liquides à température ambiante tels que le toluène et le xylène ;
- les aldéhydes liquides à température ambiante tels que le benzaldéhyde, l'acétaldéhyde
- leurs mélanges.

Le solvant organique peut être présent dans la composition de l'invention à une teneur allant de 0,01 à 99% en poids, de préférence de 50 à 99% en poids pour une application vernis à ongles, par rapport au poids total de la composition.

Lorsque la composition associable comprend une phase aqueuse, celle-ci peut comprendre de l'eau, une eau florale telle que l'eau de bleuet et/ou une eau minérale telle que l'eau de VITTEL, l'eau de LUCAS ou l'eau de LA ROCHE POSAY et/ou une eau thermale.

La phase aqueuse peut également comprendre des solvants autres que l'eau comme par exemple les alcools primaires tels que l'éthanol et l'isopropanol, les glycols tels que le propylène glycol, le butylène glycol, le dipropylène glycol, le diéthylène glycol, les éthers de glycol tel que les alkyl(C₁-C₄)éther de mono, di- ou tripropylène glycol, mono, di- ou triéthylène glycol, et leurs mélanges.

La phase aqueuse peut également comprendre au moins un colorant hydrosoluble choisi parmi les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, et leurs mélanges.

La phase aqueuse peut également comprendre tout composé compatible avec une phase aqueuse (hydrosoluble ou hydrodispersible) tels que des gélifiants, des polymères filmogènes, des épaississants, des tensioactifs, conservateurs, des dispersions de pigments.

Lorsque la composition associable est un vernis à ongles, la phase aqueuse peut être constituée essentiellement d'eau ou d'un mélange hydroalcoolique comprenant notamment des monoalcools en C₁-C₅ ou des glycols en C₂-C₈.

De préférence, la phase aqueuse est présente dans les compositions de l'invention à une teneur allant 0,01 à 99% en poids, de préférence de 0,1 à 80% en poids, par rapport au poids total de la composition. Lorsque la composition selon l'invention est une poudre, par exemple pour le visage, la phase aqueuse est de préférence présente à une teneur allant de 0,01 à 30% en poids, par rapport au poids total de la composition.

La composition associable peut également comprendre au moins un composé amphiphile, c'est-à-dire un composé comprenant à la fois une partie lipophile (partie apolaire) et une partie hydrophile (partie polaire) et pouvant s'adsorber à une surface ou une interface. De tels composés sont par exemple les émulsionnants et les co-émulsionnants.

Les émulsionnants et les coémulsionnants utilisés dans la composition selon l'invention lorsqu'elle est sous forme d'émulsion sont choisis parmi ceux classiquement utilisés dans les domaines cosmétique et dermatologique. L'émulsionnant et le coémulsionnant peuvent être présents, dans la composition, en une proportion allant de préférence de 0,3 à 30 % en poids, et mieux de 0,5 à 20 % en poids par rapport au poids total de la composition.

Comme tensioactif H/E, on peut citer notamment (CTFA) : le cétéaryl-glucoside, le PEG-40 stéarate, le sorbitan tristéarate, le sorbitan stéarate, le polysorbate 60, le mélange sorbitan stéarate/sucrose cocoate, le mélange de glycéryl stéarate/PEG-100 stéarate, le PEG-400, le stéarate de glycéryle, le mélange de PEG-6/PEG-32/glycol stéarate. Comme tensioactif E/H, on peut citer notamment le mélange polyglycéryl-4 isostéarate/ cétyldiméthicone copolyol/ hexyl laurate et le mélange Mineral oil/petrolatum/ozokérite/glycéryl oléate/alcool de lanoline.

La composition associable peut également comprendre en outre au moins un composé pulvérulent. Les composés pulvérulents peuvent être choisis parmi les pigments et/ou les nacres et/ou les charges et/ou leurs mélanges habituellement utilisés dans les compositions cosmétiques. Avantageusement, les composés pulvérulents représentent de 0,1 à 99,9%, de préférence de 60 à 99,9%, en poids, par rapport au poids total de ladite composition. Lorsque ladite composition selon l'invention est une poudre, par exemple pour le visage, les composés pulvérulents représentent de préférence de 80 à 99,9% en poids, par rapport au poids total de la composition.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être minérales ou organiques, lamellaires ou sphériques. On peut citer le talc, le mica, la silice, le kaolin, les poudres de Nylon (Orgasol de chez Atochem ou Nylon 12), de poly-p-alanine et de polyéthylène, le Téflon, l'oxychlorure de bismuth, la lauroyl-lysine, l'amidon, le nitrure de bore, les poudres de polymères dé tétrafluoroéthylène, les microsphères creuses telles que l'Expancel (Nobel Industrie), le polytrap (Dow Coming) et les microbilles de résine de silicone (Tospearls de Toshiba, par exemple), le carbonate de calcium précipité, le carbonaté et l'hydro-carbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition associable peut comprendre, en outre, tout composé usuellement utilisé dans de telles compositions, tel que des épaississants, des antioxydants, des parfums, des conservateurs, des tensioactifs, des actifs.

Comme actifs cosmétiques, dermatologiques, hygiéniques, utilisables dans la composition de l'invention, on peut citer les hydratants, vitamines, acides gras essentiels, sphingolipides, filtres solaires. Ces actifs sont présents en quantité généralement utilisée dans les poudres. En particulier, ils sont présents à raison de 0,001 à 20 % du poids total de la composition.

Bien entendu l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

Les compositions selon l'invention peuvent être préparées de manière usuelle par l'homme du métier. Elles peuvent se présenter sous forme d'une poudre libre ou compacte, d'un gel anhydre ou d'un gel aqueux, d'une émulsion E/H ou H/E ou d'une émulsion multiple. Elles peuvent se présenter par exemple sous la forme d'un stick ou de coupelle utilisable par prise au doigt ou à l'éponge, de crème : en particulier, elles trouvent une application en tant que blush, fard à joues ou à paupières, poudre à lèvres, poudre de maquillage du visage ou du corps ou encore de poudre déodorante, rouge à lèvres, fond de teint, mascara, eye-liner, anticernes. Elles peuvent aussi se présenter sous forme d'une poudre libre, notamment comme poudre à joues, de maquillage du visage ou du corps ou de poudre déodorante. Elles peuvent aussi se présenter sous forme d'une solution ou d'une dispersion et trouver une application en tant que vernis à ongles.

Ces compositions à application topique peuvent constituer notamment une composition cosmétique, hygiénique de protection, de traitement ou de soin pour le visage, pour le cou, pour les mains, pour les phanères ou pour le corps (par exemple poudre de soin anhydre parfumée ou non, poudre solaire), une composition de maquillage (par exemple gel de maquillage) ou une composition de bronzage artificiel.

Dans un mode particulier de réalisation de l'invention, la composition associable selon l'invention comprend au moins 80% de composés pulvérulents. On obtient alors un produit présentant une texture particulière, sous forme de gomme c'est-à-dire d'un produit solide, qui ne se déforme pas sous son propre poids.

Lorsque la composition associable est sous forme de gomme, cette gomme peut se présenter dans une coupelle et peut être utilisée comme une composition gommante et/ou de maquillage. Le prélèvement du produit s'effectue de préférence avec le doigt, en frottant plusieurs fois ce dernier sur la surface de la gomme : on détache ainsi des mini-rouleaux de gomme que l'on peut ensuite appliquer sur la peau. En frottant ces mini-rouleaux sur la peau, on gomme les cellules mortes de cette dernière, on masse la peau sans aucun effet agressif. La composition selon l'invention est alors utilisée pour ses propriétés gommantes. Par ce geste, on répartit également de façon homogène les particules colorées contenues dans la composition selon l'invention. La peau est ainsi à la fois douce et colorée. La composition selon l'invention est donc également utilisée pour maquiller la peau. Enfin, en dernier lieu, on élimine les mini-rouleaux, qui sont solides, simplement par effleurement de la peau avec la main et on obtient ainsi une peau à la fois douce et maquillée. On ne ressent aucun effet de collant ou de gras sur la peau.

Les compositions selon l'invention peuvent être utilisées seules, en application sur la peau du corps ou du visage ou sur les muqueuses. Ces compositions présentent une très bonne tenue et de très bonnes propriétés de sans transfert dès l'application. Par ailleurs, au cours du temps, la sueur ou le sébum sécrété par la peau interagit avec le matériau pro-adhésif, renforçant encore la tenue de la composition associable et ses propriétés de sans transfert.

Dans une autre forme de réalisation de l'invention, la composition associable est utilisée distinctement et en association avec une composition associée comprenant un composé diffusant.

Ce composé diffusant est tel qu'il forme, couplé avec le matériau pro-adhésif, un système présentant des propriétés adhésives appelé ci-après et dans toute la demande « système adhésif ». Le composé diffusant développe le pouvoir collant du matériau pro-adhésif en se diffusant au sein du réseau polymérique dudit matériau, de référence lors de l'utilisation sur la peau pour obtenir l'effet cosmétique cherché. Il est important que le matériau pro-adhésif et le composé diffusant ne soient pas en contact avant l'utilisation, au sein du conditionnement, de façon à ne pas créer d'effet collant au toucher. Dans le cas d'une composition associable pulvérulente comprenant ledit matériau pro-adhésif, il y a en plus un risque d'instabilité de la composition, c'est-à-dire que, lors d'un contact entre le matériau pro-adhésif et le composé diffusant, les poudres risquent fortement de s'agglomérer.

Le « système adhésif » selon l'invention répond aux conditions suivantes :
- G'_{SA}(2 Hz, 35°C) ≥ 10³ Pa,
- 10⁸ Pa ≥ G'_{SA}(35°C), de préférence 10⁷ Pa ≥ G'_{SA}(35°C), et
- G'_{SA}(2 10⁻² Hz, 35°C) ≤ 3 10⁵ Pa,
dans lesquelles :
- G'_{SA}(2 Hz, 35°C) est le module élastique de cisaillement du système adhésif, mesuré à la fréquence de 2 Hz et à la température de 35°C,
- G'_{SA}(35°C) est le module élastique de cisaillement du système adhésif, à la température de 35°C,
- G'_{SA}(2 10⁻² Hz, 35°C) est le module élastique de cisaillement du système adhésif, mesuré à la fréquence de 2 10⁻² Hz et à la température de 35°C.

Dans une forme préférée de réalisation de l'invention, le système adhésif répond également à la condition suivante :
- G"_{SA}/G'_{SA} (0,2 Hz, 35°C) ≥ 0,35,
dans laquelle :
- G"_{SA}(0,2 Hz, 35°C) est le module visqueux de cisaillement dudit système adhésif, mesuré à la fréquence de 0,2 Hz et à la température de 35°C,
- G'_{SA}(0,2 Hz, 35°C) est le module élastique de cisaillement dudit système adhésif, mesuré à la fréquence de 0,2 Hz et à la température de 35°C.

Dans une forme préférée de l'invention, on a :
- G'_{SA}(2 Hz, 35°C) ≥ 10⁴ Pa.

Dans une autre forme préférée de l'invention, on a :
- G'_{SA}(2 10⁻² Hz, 35°C) ≤ 5 10⁴ Pa.

De préférence encore, les systèmes adhésifs selon l'invention répondent aux quatre conditions suivantes :
- G'_{SA}(2 Hz, 35°C) ≥ 10⁴ Pa, et
- 10⁸ Pa ≥ G'_{SA}(35°C), de préférence 10⁷ Pa ≥ G'_{SA}(35°C), et
- G'_{SA}(2 10⁻² Hz, 35°C) ≤ 5 10⁴ Pa, et
- G"_{SA}/G'_{SA} (0,2 Hz, 35°C) ≥ 0,35.

Le composé diffusant peut être toute huile de faible masse moléculaire, par exemple de masse moléculaire inférieure ou égale à 20000. Ce composé diffusant peut être choisi parmi les polyisobutylènes, les lanolines, les résines hydrocarbonées aliphatiques ou aromatiques, les résines terpéniques et leurs mélanges, les résines de type « résines tackifiantes » hydrocarbonées aliphatiques ou aromatiques; il est au moins partiellement compatible avec le matériau pro-adhésif.

Comme composé diffusant convenant particulièrement bien à la présente invention, on peut citer les polyisobutylènes de masse molaire relative Mv inférieure ou égale à 10 000 comme les polyisobutylènes de masse molaire 455 à 2000 vendus sous la dénomination commerciale « Napvis » par la société BP Chemicals ou les polyisobutylènes vendus sous la dénomination « Parleam » par les Ets B. Rossow et Cie, ou encore les polyisobutylènes vendus sous la dénomination commerciale « Vistanex LM » par la société Exxon et leurs mélanges.

Ces composés diffusants conviennent particulièrement en association avec les matériaux pro-adhésifs préférés de l'invention comme les polyisobutylènes de masses molaires relatives supérieure à 150000 de la gamme Oppanol de BASF, notamment les polyisobutylènes de masses molaires relatives Mv 200 000, 400 000 et 1 110 000 vendus sous les dénominations commerciales respectives « Oppanol B 30 SF », « Oppanol B 50 SF » et « Oppanol B 100 » par la société BASF, les polyisobutylènes de masses molaires relative (moyenne viscosimétrique) comprises entre 900 000 et 2 200 000 vendus sous la dénomination commerciale « Vistanex MM » par la société Exxon, et leurs mélanges.

De préférence, le rapport pondéral entre le matériau pro-adhésif et le composé diffusant va de 0,01 à 10, de préférence encore de 0,05 à 2 et encore mieux de 0.1 à 2.

Comme associations « matériau pro-adhésif + composé diffusant » préférées de l'invention on peut également citer les matériaux pro-adhésifs comme les polymères blocs de type styrène-butadiène-styrène, styrène-(ethylène-butylène)-styrène, styrène-isoprène-styrène comme ceux vendus sous la dénomination commerciale « Kraton » de Shell Chemical Co. ou « Vector » d'Exxon, associés à des résines tackifiantes hydrocarbonées aliphatiques ou aromatiques comme les résines vendues sous la dénomination « Piccotac », « Hercotac » de Hercules ou « Escorez » d'Exxon.

Outre le composé diffusant, la « composition associée » peut comprendre tout support cosmétiquement acceptable tel que défini dans la présente demande pour la composition associable selon l'invention. Elle peut également se présenter sous toutes les formes, gels, émulsions, sticks déjà définies dans la présente demande pour la composition selon l'invention.

L'association de la composition associable et de la composition associée forme un kit utilisable pour tout produit cosmétique de maquillage. On obtient après application sur la peau des deux compositions un produit cosmétique présentant une très bonne tenue et/ou de très bonnes propriétés sans transfert.

L'invention est illustrée plus en détail dans les exemples suivants.

### EXEMPLE 1 :

La Demanderesse a réalisé la base cosmétique A suivante sous la forme d'une émulsion E/H :

### Composition A :

| *Phase A* : | |
|---|---|
| - isostéarate de sorbitane | 4,2 % |
| - conservateur | 0,2 % |
| *Phase B1 :* | |
| - isobutylène hydrogéné | 12 % |

| *Phase B2* : | |
|---|---|
| - polyisobutylène de masse moléculaire 1 110 000 vendu sous la dénomination commerciale « Oppanol B 100 » par la société BASF (matériau proadhésif) | 2 % |
| - isododécane | 12 % |

| *Phase C :* | |
|---|---|
| - eau | 70,7% |
| - sulfate de magnésium | 0,7 % |
| - conservateur | 0,2 % |

Le polyisobutylène de masse molaire 1 110 000 de cette composition présente une valeur de G'(35°C) ≥ 6 10⁴ Pa pour toute fréquence allant de 2 10⁻² à 2 Hz.

Cette émulsion a été réalisée selon les méthodes classiques de préparation des émulsions E/H. On a d'abord préparé la phase A par mélange. Puis on a ajouté la phase B1 sous agitation en chauffant. La phase B2 est ajoutée après avoir préalablement dissous le polyisobutylène dans l'isododécane en chauffant. Il en résulte une phase huileuse qui est chauffée pendant 5 min à 80°C. Ce mélange est refroidi à 20°C. Tous les composés de la phase C sont mélangés puis chauffés jusqu'à dissolution du conservateur. Ensuite, la phase C est refroidi à 20°C puis ajouté lentement au mélange A+B1+B2.

La Demanderesse a ensuite réalisé la « composition associée » B suivante qui est une poudre de visage.

### Composition B :

| | |
|---|---|
| - acétyl tributyl citrate | 8% |
| - oxyde de fer (rouge) | 5% |
| - talc | 79% |
| - polyisobutylène de masse moléculaire 455 vendu sous la dénomination commerciale « Napvis D 07 » par la société BP Chemicals (composé diffusant) | 8 % |

Le système adhésif constitué du matériau pro-adhésif de la composition A et du composé diffusant de la « composition associée » B dans un rapport de 0,25 a pour caractéristiques :
- G'_{SA}(2 Hz, 35°C) = 7 10³ Pa, et
- G'_{SA}(2 10⁻² Hz, 35°C) = 2 10³ Pa
- G"_{SA}/G'_{SA} (0,2 Hz, 35°C) = 0,35

On applique, par exemple sur le visage d'une personne, la composition A. Puis on applique ensuite, sur la composition A, la composition B. Le produit de maquillage obtenu présente une tenue remarquable et transfère très peu.

### EXEMPLE 2 :

La demanderesse a réalisé le kit suivant comprenant :
- une base i°) pour ongles comprenant le matériau proadhésif,
- une composition de vernis à ongles ii°) contenant le composé diffusant.

### Base i :

| | |
|---|---|
| - polyisobutylène de masse moléculaire 1 110 000 vendu sous la dénomination commerciale « Oppanol B 100 » par la société BASF (matériau proadhésif) | 5 % |
| - heptane | qsp 100 % |

Le polyisobutylène de masse molaire 1 110 000 de cette composition présente une valeur de G'(35°C) ≥ 6 10⁴ Pa pour toute fréquence allant de 2 10⁻² à 2 Hz.

### Vernis à ongles ii):

| | |
|---|---|
| - nitrocellulose | 12 % |
| - N-éthyl o,p-toluène-sulfonamide | 4 % |
| - acétyl citrate de tri-butyle | 4 % |
| - pigment | 1% |
| - hectorite | 1.2 % |
| - alcool isopropylique | 5.1 % |
| - polyisobutylène de masse moléculaire 455 vendu sous la dénomination commerciale « Napvis D 07 » par la société BP Chemicals (composé diffusant) | 10 % |
| - acétate d'éthyle, acétate de butyle | qsp 100 % |

Le système adhésif constitué du matériau pro-adhésif de la base i°) et du composé diffusant du vernis à ongles ii°) (« composition associée » selon l'invention) dans un rapport de 0,5 a pour caractéristiques :
- G'_{SA}(2 Hz, 35°C) = 3 10⁴ Pa, et
   G'_{SA}(2 10⁻² Hz, 35°C) ≤ 2 10⁴ Pa

On applique sur les ongles ou les faux ongles la base i°). On applique ensuite le vernis ii°). Le produit obtenu a une tenue remarquable.

## Revendications

1. Kit de maquillage comprenant :
- i°) une composition dite associable comprenant, dans un support cosmétiquement acceptable, au moins un matériau pro-adhésif répondant à la condition suivante :
- 10⁸ Pa ≥ G'(35°C) ≥ 2 10⁴ Pa pour toute fréquence allant de 2 10⁻² à 2 Hz, de préférence :
- 10⁷ Pa ≥ G'(35°C) ≥ 2 10⁴ Pa pour toute fréquence allant de 2 10⁻² à 2 Hz,
dans laquelle :
- G'(35°C) est le module élastique de cisaillement dudit matériau pro-adhésif, mesuré. à la température de 35°C,
ledit matériau pro-adhésif étant choisi parmi les polyisobutylènes,
et,
- ii°) une « composition associée » comprenant, dans un support cosmétiquement acceptable, au moins un composé diffusant choisi parmi les huiles de masse moléculaire inférieure ou égale à 20000 tel que le système adhésif « matériau pro-adhésif + composé diffusant » répond aux conditions suivantes :
- G'_{SA}(2 Hz, 35°C) ≥ 10³ Pa,
- 10⁸ Pa ≥ G'_{SA}(35°C), de préférence 10⁷ Pa ≥ G'_{SA}(35°C), et
- G'_{SA}(2 10⁻² Hz, 35°C) ≤ 3 10⁵ Pa,
dans lesquelles :
- G'_{SA}(2 Hz, 35°C) est le module élastique de cisaillement du système adhésif, mesuré à la fréquence de 2 Hz et à la température de 35°C,
- G'_{SA}(35°C) est le module élastique de cisaillement du système adhésif, à la température de 35°C, pour toute fréquence comprise entre 2 10⁻² et 2 Hz
- G'_{SA}(2 10⁻² Hz, 35°C) est le module élastique de cisaillement du système adhésif, mesuré à la fréquence de 2 10⁻² Hz et à la température de 35°C.

2. Kit selon la revendication 1, **caractérisé en ce que** le matériau pro-adhésif est un polyisobutylène présentant une masse molaire relative Mv supérieure ou égale à 150 000.

3. Kit selon la revendication 1 ou 2, **caractérisé en ce que** le matériau pro-adhésif est un polyisobutylène présentant une masse molaire relative Mv est comprise entre 900 000 et 2 200 000

4. Kit selon l'une quelconque des revendications précédentes, **caractérisée en ce que** le composé diffusant est choisi parmi les polyisobutylènes, les lanolines, les résines hydrocarbonées aliphatiques ou aromatiques, les résines terpéniques, les résines tackifiantes hydrocarbonées aliphatiques ou aromatiques, et leurs mélanges.

5. Kit selon l'une quelconque des revendications précédentes, **caractérisé en ce que** le composé diffusant est choisi parmi les polyisobutylènes.

6. Kit selon l'une quelconque des revendications précédentes, **caractérisé en ce que** le composé diffusant est un polyisobutylène de masse molaire relative Mv allant de 455 à 2000.

7. Kit selon l'une quelconque des revendications précédentes, **caractérisé en ce que** la composition associable et/ou associée comprend une huile volatile.

8. Kit selon l'une quelconque des revendications précédentes, **caractérisé en ce que** la composition associable et/ou associée comprend une phase solvant organique.

9. Kit selon l'une quelconque des revendications précédentes, **caractérisé en ce que** la composition associable et/ou la composition associées comprennent en outre un composé pulvérulent choisi parmi les pigments et/ou les nacres et/ou les charges et/ou leur mélange.

10. Procédé non thérapeutique de maquillage de la peau, des muqueuses, des semi-muqueuses et/ou des phanères d'humain, consistant à appliquer sur lesdites peau, muqueuses, semi-muqueuses et/ou phanères d'humain la composition associée avant ou après la composition dite associable du kit selon l'une des revendications précédentes.

11. Utilisation du kit de maquillage selon l'une quelconque des revendications 1 à 9 pour obtenir un maquillage ayant une bonne tenue et/ou sans transfert.

## Claims

1. Makeup kit comprising:
i) a "combinable" composition comprising, in a cosmetically acceptable support, at least one pro-adhesive material that satisfies the following condition:
- 10⁸ Pa ≥ G' (35°C) ≥ 2 × 10⁴ Pa for any frequency ranging from 2 × 10⁻² to 2 Hz, preferably:
- 10⁷ Pa ≥ G'(35°C) ≥ 2 × 10⁴ Pa for any frequency ranging from 2 × 10⁻² to 2 Hz,
in which:
- G' (35°C) is the elastic shear modulus of the said pro-adhesive material, measured at a temperature of 35°C,
the said pro-adhesive material being chosen from polyisobutylenes, and
ii) a "combined composition" comprising, in a cosmetically acceptable support, at least one diffusing compound chosen from oils with a molecular mass of less than or equal to 20 000, such that the adhesive system of "pro-adhesive material + diffusing compound" satisfies the following conditions:
- G'_{AS}(2 Hz, 35°C) ≥ 10³ Pa,
- 10⁸ Pa ≥ G'_{AS}(35°C), preferably 10⁷ Pa ≥ G'_{AS}(35°C), and
- G'_{AS}(2 × 10⁻² Hz, 35°C) ≤ 3 × 10⁵ Pa,
in which:
- G'_{AS}(2 Hz, 35°C) is the elastic shear modulus of the adhesive system, measured at a frequency of 2 Hz and at a temperature of 35°C,
- G'_{AS}(35°C) is the elastic shear modulus of the adhesive system, at a temperature of 35°C, for any frequency of between 2 × 10⁻² and 2 Hz, and
- G'_{AS} (2 × 10⁻² Hz, 35°C) is the elastic shear modulus of the adhesive system, measured at a frequency of 2 × 10⁻² Hz and at a temperature of 35°C.

2. Kit according to Claim 1, **characterized in that** the pro-adhesive material is a polyisobutylene with a relative molar mass Mv of greater than or equal to 150 000.

3. Kit according to Claim 1 or 2, **characterized in that** the pro-adhesive material is a polyisobutylene with a relative molar mass Mv of between 900 000 and 2 200 000.

4. Kit according to any one of the preceding claims, **characterized in that** the diffusing compound is chosen from polyisobutylenes, lanolins, aliphatic or aromatic hydrocarbon-based resins, terpenic resins, and aliphatic or aromatic hydrocarbon-based tackifying resins, and mixtures thereof.

5. Kit according to any one of the preceding claims, **characterized in that** the diffusing compound is chosen from polyisobutylenes.

6. Kit according, to any one of the preceding claims, **characterized in that** the diffusing compound is a polyisobutylene with a relative molar mass Mv ranging from 455 to 2000.

7. Kit according to any one of the preceding claims, **characterized in that** the combinable and/or combined composition comprises a volatile oil.

8. Kit according to any one of the preceding claims, **characterized in that** the combinable and/ or combined composition comprises an organic solvent phase.

9. Kit according to any one of the preceding claims, **characterized in that** the combinable composition and/or the combined composition also comprise a pulverulent compound chosen from pigments and/or nacres and/or fillers and/or a mixture thereof.

10. Non-therapeutic process for making up human skin, mucous membranes, semi-mucous membranes and/or integuments, which consists in applying to the said human skin, mucous membranes, semi-mucous membranes and/or integuments the combined composition before or after the "combinable" composition of the kit according to any one of the preceding claims.

11. Use of the makeup kit according to any one of Claims 1 to 9 for obtaining a makeup result that shows good staying power and/or that is transfer-resistant.

## Patentansprüche

1. Kit zum Schminken, enthaltend:
- i°) eine kombinierbar genannte Zusammensetzung, die in einem kosmetisch akzeptablen Träger zumindest ein Pro-Adhäsiv enthält, das die folgende Bedingung erfüllt:
- 10⁸ Pa ≥ G' (35 °C) ≥ 2· 10⁴ Pa bei jeder Frequenz von 2· 10⁻² bis 2 Hz, vorzugsweise:
- 10⁷ Pa ≥ G' (35 °C) ≥ 2· 10⁴ Pa bei jeder Frequenz von 2·10⁻² bis 2 Hz,
wobei:
- G' (35 °C) der elastische Speichermodul des Pro-Adhäsivs, gemessen bei 35 °C, ist, und
das Pro-Adhäsiv unter den Polyisobutylenen ausgewählt ist,
und
- ii°) eine « kombinierte Zusammensetzung », die in einem kosmetisch akzeptablen Träger zumindest eine diffundierende Verbindung, die unter den Ölen mit einer Molmasse kleiner oder gleich 20000 ausgewählt ist, enthält, so dass das haftende System « Pro-Adhäsiv + diffundierende Verbindung » die folgende Bedingungen erfüllt:
- G'_{SA}(2 Hz, 35 °C) ≥ 10³ Pa,
- 10⁸ Pa ≥ G'_{SA}(35 °C), vorzugsweise 10⁷ Pa ≥ G'_{SA}(35 °C), und
- G'_{SA}(2· 10⁻² Hz, 35 °C) ≤ 3· 10⁵ Pa,
wobei:
- G'_{SA}(2 Hz, 35 °C) der elastische Speichermodul des haftenden Systems ist, der bei einer Frequenz von 2 Hz und einer Temperatur von 35 °C gemessen wird,
- G'_{SA}(35 °C) der elastische Speichermodul des haftenden Systems bei einer Temperatur von 35 °C und jeder Frequenz von 2· 10⁻² bis 2 Hz ist,
- G'_{SA}(2· 10⁻² Hz, 35 °C) der elastische Speichermodul des haftenden Systems ist, der bei einer Frequenz von 2.10⁻² Hz und einer Temperatur von 35 °C gemessen wird.

2. Kit nach Anspruch 1, **dadurch gekennzeichnet, dass** das Pro-Adhäsiv ein Polyisobutylen mit einer relativen Molmasse Mv größer oder gleich 150 000 ist.

3. Kit nach Anspruch 1 oder 2, **dadurch gekennzeichnet, dass** Pro-Adhäsiv ein Polyisobutylen mit einer relativen Molmasse Mv von 900 000 bis 2 200 000 ist.

4. Kit nach einem der vorhergehenden Ansprüche, **dadurch gekennzeichnet, dass** die diffundierende Verbindung unter Polyisobutylenen, Lanolinen, aliphatischen oder aromatischen Kohlenwasserstoffharzen, Terpenharzen, klebrigmachenden aliphatischen oder aromatischen Kohlenwasserstoffharzen und deren Gemischen ausgewählt ist.

5. Kit nach einem der vorhergehenden Ansprüche, **dadurch gekennzeichnet, dass** die diffundierende Verbindung unter den Polyisobutylenen ausgewählt ist.

6. Kit nach einem der vorhergehenden Ansprüche, **dadurch gekennzeichnet, dass** die diffundierende Verbindung ein Polyisobutylen mit einer relativen Molmasse Mv von 455 bis 2000 ist.

7. Kit nach einem der vorhergehenden Ansprüche, **dadurch gekennzeichnet, dass** die kombinierbare und/oder die kombinierte Zusammensetzung ein flüchtiges Öl enthalten/enthält.

8. Kit nach einem der vorhergehenden Ansprüche, **dadurch gekennzeichnet, dass** die kombinierbare und/oder die kombinierte Zusammensetzung eine organische Lösemittelphase enthalten/ enthält.

9. Kit nach einem der vorhergehenden Ansprüche, **dadurch gekennzeichnet, dass** die kombinierbare und/oder die kombinierte Zusammensetzung zusätzlich eine pulverförmige Verbindung enthalten/enthält, die unter den Pigmenten und/oder Perlglanzpigmenten und/ oder Füllstoffen und/ oder deren Gemischen ausgewählt ist.

10. Nichttherapeutisches Verfahren zum Schminken der Haut, der Schleimhäute, der Semischleimhäute und/oder der Hautanhangsgebilde des Menschen, das darin besteht, die kombinierte Zusammensetzung vor oder nach der so genannten kombinierbaren Zusammensetzung des Kits nach einem der vorhergehenden Ansprüche auf der Haut, den Schleimhäuten, den Semischleimhäuten und/oder den Hautanhangsgebilden des Menschen anzuwenden.

11. Verwendung des Kits zum Schminken nach einem der Ansprüche 1 bis 9, um eine Schminke mit einem guten Halt und/oder ohne Übertragung zu erhalten.
